# EUROPEAN PATENT APPLICATION

(11) **EP 2 270 514 A1**
(43) Date of publication of application: **05.01.2011**
(21) Application number: 09164592.9
(22) Date of filing: 03.07.2009
(51) Int. Cl.: G01N 35/00, G01N 33/49, G01N 33/80

(54) **Method for storing and tracing of manual blood typing analyses**

(71) Applicant: VidimSoft bvba, 8490 Jabbeke (BE)
(72) Inventor: Muylle, Jean-Pierre, 8870, Kachtem (BE)
(74) Representative: BiiP cvba

(57) **Abstract**

The present invention generally relates to a hardware and software platform for clinical and private laboratories, more specifically for the manual blood group and antibody analyses.

The present invention relates to a carrier plate comprising a plurality of test cups for performing an analyses of a blood sample, optionally a full blood typing analyses. The present invention also relates to a method for automated storing and tracing of said blood sample results.

## Description

### Field of the invention

The present invention generally relates to a hardware and software platform for clinical and private laboratories, more specifically for manual blood group and antibody analyses.

The present invention relates to a carrier plate comprising a plurality of test cups for performing a manual analyses of a blood sample, optionally a full blood typing analyses. The present invention also relates to a method for automated storing and tracing of said manual blood typing analyses.

### Background of the invention

Blood typing is a routine procedure in modern medicine. Immunological agglutination reactions are used for identifying various kinds of blood types and for detecting various kinds of antibodies and antigens in blood samples and other aqueous solutions. In a conventional manual procedure, a sample of red blood cells is mixed with serum or plasma in test tubes, slides or opaline plates, and the mixture may then be incubated and centrifuged. Various reactions either occur or do not occur depending on, for example, the blood type of the red blood cells or whether certain antibodies are present in the blood sample. Typically, these reactions manifest themselves as clumps of cells or particles with antigens and antibodies on their surfaces, referred to as agglutinates.Thus, the absence of any such clumps indicates that no reaction has occurred; and the presence of such clumps indicates that a reaction has occurred, with the size and amount of such clumps being a quantitative indicator of the level or concentration in the sample, or an indicator of the reaction strength, affinity of the complex for which the blood sample was tested.

Column Agglutination Technology (CAT) is a recent agglutination technique. It may be defined as the analyses of blood and blood products utilizing filtration as a means of separating agglutinated, precipitated, absorbed, or adsorbed particulate components from non-reactive components for immunoassay applications. In this method, gel or glass bead microparticles are contained within a small column, referred to as a microcolumn. A reagent such as anti-IgG is dispensed in a diluent in the microcolumn and test red blood cells are placed in a reaction chamber above the column. The column, which is typically one of a multitude of columns formed in a transparent cassette, is centrifuged.The centrifuging accelerates the reaction, if any, between the reagent and the blood cells, and also urges the cells toward the bottom of the column. The glass beads or gel in the microcolumn act as a filter, however, and resist or impede downward movement of the particles in the column. As a result, the nature and distribution of the particles in the microcolumn after centrifuging provides a visual indication of whether any agglutination reaction occurred in the microcolumn, and if so, of the strength of that reaction.

In particular, if no agglutination reaction occurs, then all or virtually all of the red blood cells in the microcolumn pass downward, during centrifuging, to the bottom of the column and form a pellet at that bottom. If there is a very strong reaction between the reagent and the red blood cells, virtually all of the red blood cells agglutinate, and large agglutinates form at the top of the microcolumn, above the gel or glass beads contained therein. The gel or glass beads prevent the agglutinates from passing, during centrifuging, to the bottom of the column, so that after centrifuging the agglutinates remain above the gel or beads.

If there is a reaction between the reagent and the blood cells, but this reaction is not as strong as the above-described very strong reaction, then some but not all of the red blood cells agglutinate. The percentage of red blood cells that agglutinate and the size of the agglutinated particles both vary directly with the strength of the reaction. During centrifuging, the unreacted blood cells pass to the bottom of the column, and the distance that the agglutinated particles pass downward through the column depends on the size and number of those particles. Hence, the size of the pellet of red blood cells at the bottom of the microcolumn, and the extent to which the agglutinates penetrate into the gel or glass beads in the microcolumn, are both inversely related to the strength of the reaction between the reagent and the red blood cells.

With this CAT, after the desired processing steps have been performed, the microcolumn is observed, or read, by a human operator, who then classifies the reaction between the reagent and the red blood cells. Conventionally, the reaction is classified as either negative or positive by reading the height within the column. If positive, the reaction is then further classified into one of four classes depending on the strength of the reaction. Reading the reaction as such is a one-dimensional problem, i.e. the height within the column.

Well-known microtube column agglutination technologies include DiaMed-ID^{®} and Ortho BioVue^{®}, using dedicated cassettes comprising microcolumns. Said dedicated cassettes however cannot be afforded by every lab. The term "cassettes" means any type of microtube column agglutination cassette technique.

The column agglutination technique may be automated. EP0637744B1 describes a system and method for automatically reading and classifying agglutination reactions that occur within a column or tube.

Automated systems require dedicated and expensive equipment. This is not an option for almost 70% of European labs, let alone the rest of the world, that still use the traditional manual blood typing method whereby a blood typing analyses is prepared in a test tube and an agglutination pattern is present or not after centrifuging. The content of the tube is poured onto a cardboard holder which may then be used for interpretation. The cardboard holder is stored as such for later reference. When performed correctly, said techniques are less expensive than the available cassette techniques as described above.

New European guidelines (2005/61/EG) however demand that laboratoria can provide full traceability of blood typing analyses results. Traceability of results is especially very important for blood banks and blood transfusion. Automatic or semiautomatic blood typing methods guarentee a good traceability within the lab, usually high budget labs. Manual blood typing however still shows many drawbacks concerning reliability and traceability of analyses and results. Take for example the manual test tube method where the content of the test tube is poured onto a cardboard holder and stored as such for later reference. After time however, the coagulated pattern of blood on the cardboard fades or crumbles away leaving no traceability at all. As such, there is a strong need for providing reliable and traceable methods for manual blood typing analyses.

Moreover, the reality is yet more complex, since few small or medium-sized laboratories work with only one technique. Legislation in some countries (e.g. France) is such that a double testing is needed for a valid blood sample analyses. The need for providing traceability must therefor be extended to the need for providing reliable and traceable methods comprising different manual blood typing techniques in one system.

### Summary of the invention

It is an object of the present invention to provide an affordable, safe, reliable and fully traceable platform for manual blood sampling and blood typing analyses for small and medium-sized labs.

It is another object of the present invention to provide an affordable, safe, reliable and fully traceable platform for blood sampling and blood typing analyses tested by two or more manual techniques.

In a first aspect there is provided a method for automated storing of a manual blood typing analyses at least partly performed in one or more carrier plates comprising a plurality of cups, said method comprising the steps of:
a. applying a patient ID and a sample ID to said one or more carrier plates;
b. providing a mixture of blood sample and a suitable reagent in one or more cups of said carrier plates;
c. digitizing an image of said carrier plates;
d. interpreting (classifying) an agglutination reaction within said one or more cups of said carrier plates;
e. storing said patient ID, sample ID, image and interpretation in an automated system.

In one embodiment step b. optionally comprises incubating said mixture within said carrier plates.

In another embodiment the mixture of blood sample and a suitable reagent of step b. is first mixed and optionally incubated in a test tube and then poured into said one or more carrier plates.

In a further embodiment of the method said manual blood typing analyses is partly performed in one or more carrier plates and partly in one or more cassettes, said method further comprising the steps of:
a. applying said patient ID and said sample ID to said cassettes;
b. providing a mixture of blood sample and a suitable reagent in one or more tubes of said cassettes;
c. digitizing an image of said cassettes;
d. interpreting an agglutination reaction within said one or more tubes;
e. storing said patient ID, sample ID, image and interpretation in an automated system.

In one embodiment step d. comprises the step of manually validating each of said interpretations of said blood typing analyses.

In another embodiment said method comprises the step of biologically validating all interpretations for said patient ID and said sample ID.

In a second aspect there is provided a method for tracing a manual blood typing analyses at least partly performed in one or more carrier plates comprising a plurality of cups, said method comprising the steps of:
a. providing a patient ID and/or sample ID;
b. selecting a stored blood typing analyses performed as in any of the previous claims;
c. retrieving one or more of the steps performed within said automated blood typing analyses.

In a third aspect there is provided a method for performing a manual blood typing analyses in a carrier plate comprising a plurality of cups, said method comprising the steps of:
a. providing a mixture of blood sample and a suitable reagent in one or more cups of said carrier plate, and optionally incubating said mixture within said carrier plate;
b. interpreting an agglutination reaction within said one or more cups of said carrier plate;

The present invention provides a safe and traceable platform for performing manual blood typing analyses. Manual blood typing is cheap and allows the choice of suppliers (e.g. for reagents). Other advantages of the invention include a transparent cross checking by superiors, and an easy exchange of analyses results as well in the lab, as online or remote, hereby avoiding the need for a clinical biologist to be present at each remote location.

The methods of the present invention combine the advantages of known manual blood typing analyses methods, i.e. the opaline and test tube technique. Like the opaline technique, the methods of the invention are fast and do not need centrifuging or multiple test tubes, which are exactly the disadvantages of the test tube technique. This latter technique however, and even so the methods of the invention, can interprete very weak reactions (diluted suspensions) and double populations.

### Description of the invention

The present invention provides an affordable, reliable and traceable method for manual blood typing analyses. For this purpose the present invention provides a method for digitizing and interpreting the manual blood sampling technique of test tubes. Herefor the cardboard holder is "replaced" by a carrier plate comprising test cups. Each test tube is poured within a test cup of said carrier plate to visualise a possible agglutination pattern. The test plate is then scanned for further interpretation, either visually or automatically. The design of the carrier plate is such that the blood sampling analyses may be performed directly within the test cups hereby removing the use of cumbersome test tubes. The carrier plate comprises specific features that contribute to the reliability and traceability of the method of the invention.

The carrier plates of the present invention are to be distinguished from the microplates (also referred to as microtiter plates) used in automated methods as for example described in US 5,192,692. The small wells of a 96-well microplate need to be pipeted by an automated robot and the interpretation is fully computer-controlled. Many small laboratories use these microplates to perform a manual analyses. This manner of manual blood typing analyses however is not accurate and very error-prone due to the small size of the cups and the large amount of cups. Manual pipeting is very difficult in the small wells, as is the manual interpretation of the result. Moreover, since the microtiter plate comprises multiple wells, labs are seduced to perform blood typing analyses for multiple patients in one and the same microtiter plate. This must inevitably lead to errors in interpreting the column and row of the cup at stake. With microtiter plates it is further difficult to ensure that the manufacturer's instructions are followed precisely. The plates require agitation to resuspend nonagglutinated cells. It is further difficult to standardise reading of weak reactions. It is also difficult for a supervisor to monitor or confirm the reactions. The carrier plates of the present invention overcome these disadvantages.

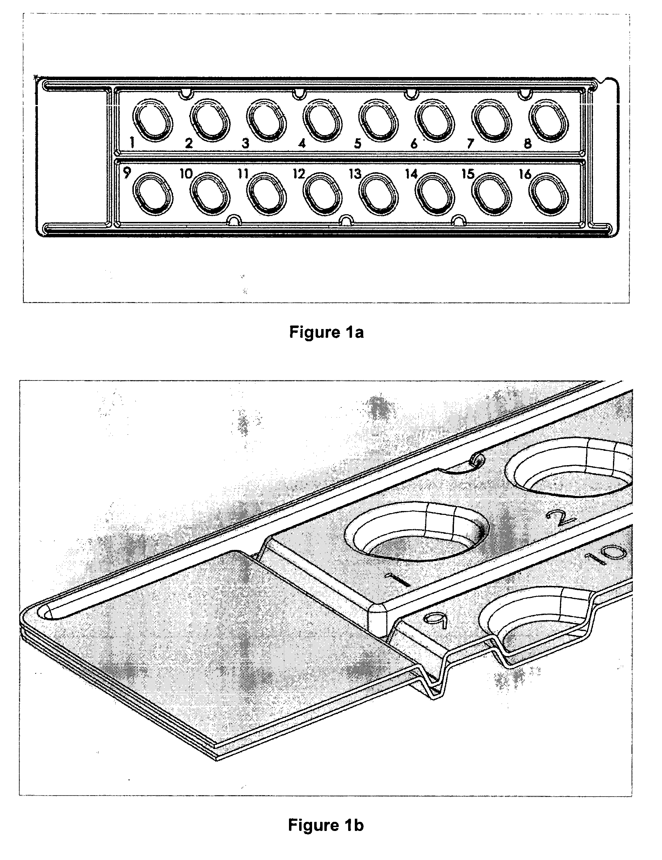

According to a first aspect of the invention there is provided a carrier plate comprising a plurality of test cups for performing a manual blood typing analyses. Figure 1 a and 1 b illustrate an embodiment of a carrier plate according to the present invention.

A full blood test comprising phenotypes, Kell and screening easily takes about 12 cups. In one embodiment the carrier plate comprises 12 cups. In a preferred embodiment the carrier plate comprises 16 cups for performing a full blood typing analyses and some other extra tests.
The carrier plate may comprise more or less cups. In one embodiment the carrier plate comprises 32 cups for performing a double blood typing analyses for one patient. In yet another embodiment the carrier plate comprises 24 cups.

In one embodiment the cups of the carrier plate are numbered, as shown in Figures 1 a and 1 b.

The carrier plate comprises a reference indication for uniquely positioning the carrier in relation to a second reference. In one embodiment said second reference is a scanner or a holding means within a scanner. In another embodiment said second reference is provided on a paper, such as e.g. a Device Work Document. The carrier plate is uniquely positioned on the work document with the carrier plate reference indication positioned on the work document reference indication. The work document comprises corresponding cup numbers as applied on the carrier plate. The work document preferably also comprises an indication per cup of antigens to be tested. Other indications may be added within the scope of the present invention. A work document may indicate e.g. A, B, AB, D, CTRL-D, A/T, REV-A1, REV-B, C, E, c, e, K, k, REV-A2 and REV-O, respectively at positions 1 to 16. Within the scope of the invention the position of the indications may vary. The indications improve the reliability and safety of the test, since the chance of putting a wrong antigen in a cup is minimised.

In one embodiment the carrier plate has a design as shown in Figures 1 a and 1 b. The cup design (size and concavity) is such that an appropriate agglutination reaction can be performed within the cups, but also that pipeting is made easy and the droplet falls nicely within a cup. Red blood cells have a tendency to clot to the side wall when a cup is too flat; cups with too much curvature however have a tendency towards false positives. The cups are preferably circular or oval. Manual pipeting is improved by placing the cups in a diagonal offset from the centerline.

Within the cups of the carrier plate plasma is applied to optimize the surface tension. This treatment preferably lasts up to 12 months. No change in result may be observed due to said plasma or any additives. In one embodiment said plasma is similar to that used in the wells of microplates. In another embodiment plasma and additives are applied. Suitable additives may be acrylic acid.

In one embodiment the carrier plate comprises an indication linked to patient information or a Patient ID, such as e.g. a barcode. The carrier plate preferably also comprises a sample ID of the blood sample. In another embodiment the carrier plate may further comprise a carrier plate indication specifying e.g. the lot number and fabrication date of the carrier plate.

In one embodiment the carrier plate is made of polystyrene (PST) and is washable. In another emboidment the carrier plate is made of polyethylene terephthalate (PET). In a preferred embodiment the carrier plate is made of lightweight polystyrene and is disposable.

In one aspect, the methods of the invention provide a digitisable and traceable solution for the conventional manual blood typing analyses using test tubes.

The term "blood typing analyses" comprises any analyses performed on a blood sample, e.g. on red blood cells or blood serum.
The term "manual" means that the blood typing analyses is performed manually or semi-automatically in e.g. a test tube, cassette, opaline plate or carrier plate according to the invention, whereby normally the agglutination reaction would disappear with time. Manual is to be interpreted as not fully automatic, because a fully automatic blood typing analyses is certified secure and traceable, whereas this is not the case for any other not fully automatic blood typing analyses.

In one embodiment the present invention provides a method for rapidly determining the presence or absence, of red blood cells of a given individual, of an antigen selected from C, c, E, e, and K, in a carrier plate as described above, which comprises the steps of: (a) applying a sample of red blood cells to be tested in a cup of the carrier plate; (b) mixing with said sample an antibody reagent to form a mixture; and (c) observing said mixture to determine whether agglutination does or does not occur.

Said method may optionally comprise a further incubation step, wherein the mixture is shaken according to good lab practice and incubated within the cups of said carrier plate.

One or more parameters may be tested simultaneously within one carrier plate. In another embodiment the same parameters may be tested double in two carrier plates.

In one embodiment the sample comprises whole blood. In another embodiment the sample comprises diluted blood.

Any suitable reagent may be used for performing a blood analysis in a carrier plate or a test tube.

A blood typing analyses test within the carrier plate of the present invention may be performed as follows. A suspension of red blood cells to be tested is prepared in normal group compatible serum or in the patient's own serum or plasma. Whole blood may be used without dilution, since the concentration of red blood cells in the test sample conveniently should be about 40-50%. A small volume of cell suspension is dropped in each cup of the carrier plate. A small amount of the subject antibody reagent is mixed with about double the volume of the cell suspension. The carrier plate is shaken according to good lab practice so that the red blood cell suspension and the antibody reagent are mixed. The presence or absence of agglutination is evaluated after several minutes.

In another embodiment any suitable method for manual blood typing analyses may be used, such as e.g. a cassette technique, any test tube technique and opaline technique.

According to a second aspect of the invention there is provided a method for automated storing of a manual blood typing analyses at least partly performed in a carrier plate comprising a plurality of cups, said method comprising the steps of a) applying a patient ID and a sample ID to said carrier plate; b) providing a mixture of blood sample and a suitable reagent in one or more cups of said carrier plate; c) digitizing an image of said carrier plate; d) interpreting (*classifying*) an agglutination reaction within said one or more cups of said carrier plate; and e) storing said patient ID, sample ID, image and interpretation in an automated system.

Said method may optionally comprise a further incubation step, wherein the mixture is shaken according to good lab practice and incubated within the cups of said carrier plate.

Step b) of the method may involve first preparing and optionally incubating said mixture of blood sample and a suitable reagent in a test tube and then providing the content of said test tube in a cup of said carrier plate.

In another embodiment the method of the invention provides that said manual blood typing analyses is partly performed in a carrier plate and partly in a cassette technique, said method further comprising the steps of a) applying said patient ID and said sample ID to said cassette; b) providing a mixture of blood sample and a suitable reagent in one or more tubes of said cassette technique; c) digitizing an image of said cassette; d) interpreting an agglutination reaction within said one or more tubes; and e) storing said patient ID, sample ID, image and interpretation in an automated system.

The platform supports the traditional manual technique (e.g. tubes and opaline), the cassette technique (e.g. Diamed^{®},Ortho^{®}, BioRad^{®} and Grifols^{®}), and the carrier plate technique as described herein.

In a further aspect the present invention provides a method for tracing a manual blood typing analyses at least partly performed in a carrier plate comprising a plurality of cups, said method comprising the steps of: a) providing a patient ID and/or sample ID; b) selecting a stored blood typing analyses performed as in any of the previous claims; and c) retrieving one or more of the steps performed within said automated blood typing analyses. The term "tracing" means retrieving all individual steps performed within the manual blood typing analyses.

The system of the present invention consists of a hardware and software platform for storing and tracing the results of a blood typing analyses. The platform realizes a fully traceable and safe digitalisation and interpretation of blood typing analyses. The image is presented on screen and delivers a correct result ready for validation.

The platform supports the traditional manual technique (e.g. tubes and opaline), the cassette technique (e.g. Diamed^{®},Ortho^{®}, BioRad^{®} and Grifols^{®}), and the carrier plate technique as described herein. Results are managed, controlled and printed out with highly secured software. All techniques can be combined in order to have a completely controlled, traced and secured result of a patient's blood sample.

Maximum security is present as fully secured barcode labels guarantee a perfect link between test, carrier plate, sample and patient.

In one embodiment the hardware platform of the present invention comprises a computer, a scanner and preferably a hand labeler. The computer comprises storage means for storing blood sampling analyses.

In a preferred embodiment there is provided a scanner with holding means for holding carrier plates. The indents of each carrier plate fit into the respective positions of the holding means, and as such provide a secured and unique interconnection within the scanner. As such there is no error as to how each carrier plate has to be placed within the scanner. The carrier plates are positioned within close proximity of the scanner glass for optimal scanning quality.

The scanner may digitize up to four 16 cup carrier plates at a time, whereby four images are stored. The barcode of each carrier plate is interpreted and linked to the scanned image. As such, the results of a test are linked to an individual and a date of testing. Other information may also be stored

The software platform allows for the interpretation of the scanned images of sample tests.

In one embodiment the interpreting step is performed by a lab technician visually classifying an agglutination reaction provided by an image of a cup.

In one embodiment a selection can be made from a list of tests. In another embodiment an image can be selected on any of sample ID, sample date, patient id and/or patient name, or any other information linked to the image.

An image sample consists of a screen display comprising the scanned image and additional information. The scanned image is preferably broken up into the individual cups (antigens) for easier interpretation. The lab technician (administrator) can enter an interpretation for each cup of the carrier plate. This interpretation is preferably selected from a list of possible interpretations. The adminsitrator can then command to process an overal result of the sample based on the individual cup interpretations. Finally the administrator may correct or aprove this result. As such, the automated manual blood typing analyses method is safe, reliable and traceable, however still completely controlled by a professional skilled in the art.

In an alternative embodiment, the software may automatically interprete the results of the cups by means of a machine vision algorithm. Yet an administrator manually approves or overrules the automatic result.

The software is adaptable so that the cups' specifics, such as e.g. indications per cup position and reagents, can be initialised.

In one embodiment the software platform may comprise a machine vision algorithm for automated identification of the scanned carrier plate image. A method for automated identification of a scanned carrier plate cup may comprise the steps of a) providing a spatial registration; b) detecting colour, shape and/or texture; c) interpreting a possible agglutination pattern; and d) classifying said agglutination pattern.

According to yet another aspect of the invention there is provided a method for storing and tracing of a first and second blood typing analyses for one and the same patient and sample, and for validating a consensus about the results of both. Legislation in some countries (e.g. France) is such that a double testing is needed for a valid blood sample analyses.

In one embodiment the first and second blood typing analyses are performed with the same method but different reagents, or reagents from different manufacturers, e.g. using two carrier plates of the present invention, each with different reagents from different manufacturers. In another embodiment the first and second blood typing analyses may be performed with different techniques, such as e.g. a carrier plate technique of the present invention and a cassette technique. In yet another embodiment the first and second blood typing analyses may be complementary, whereby one series of tests is performed by one method, and another series of tests by another method.

The method, more specifically the software, further allows to make a consensus between the results of the two blood typing analyses for the same patient. The results of both analyses are compared and should be identical in order to be biologically validated. Validation needs the aprovement of a supervisor.

The hardware and software platform of the present invention is also adapted to scan and interprete a column agglutination technique using BioVue^{®} cassettes.

In one embodiment the methods of the invention may be linked to or integrated with a Laboratory Information System (LIS).

In another embodiment one or more of the steps of the methods of the present invention are made remotely accessible, provided there is sufficient security for releasing patient information.

In a further aspect a computer system is provided for performing the methods as set out above.

In yet a further aspect a program is provided executable on a programmable device containing instructions, which executed performs the methods as set out above.

The overall advantage of the methods of the invention is the use of one platform (hardware and software) to perform a reliable analyses conform the European guidelines, also including double analyses for certain countries.

The present invention relates to a hardware and software platform for clinical and private laboratories, more specifically for the blood typing and antibody analyses.

It is within the scope of the present invention to apply the methods and carrier plates for other types of analyses. Herefor the carrier plate and the hardware and/or software platform may be adapted accordingly.

### Brief description of drawings

Figures 1 a and 1 b illustrate an embodiment of a carrier plate according to the present invention.
Figure 2 is a screenshot of a sample input.
Figures 3a and 3b show a screenshot of a virtual view of a distribution for an ABO/Grifols analyses.
Figures 4a and 4b show the labeling of a Grifols device.
Figures 5a and 5b show a screenshot of the virtual view of a distribution for a Groupe Adulte (Opaline) analyses.
Figures 6a and 6b show the labeling of a carrier plate.
Figure 7 shows a Device Work Document for the methods of the present invention.
Figure 8 shows a scanning holder frame comprising devices of type Grifols.
Figure 9 shows a scanning holder frame for a carrier plate device of the invention.
Figure 10a shows a screenshot of the scanned image of a Grifols cassette device.
Figure 10b shows a screenshot of a technical validation for one tube in the cassette.
Figure 11a shows a screenshot of the scanned image of a carrier plate device.
Figure 11b shows a screenshot of a technical validation for one cup in the carrier plate.
Figures 12 illustrates the graduation of results for a cassette technique.
Figures 13 illustrates the graduation of results for a carrier plate technique.
Figure 14 shows a screenshot of a physical view of a full sample test ready for biological validation.
Figure 15 shows a Sample Report.
Figure 16a and 16b shows a coagulation device according to the invention for the carrier plates of the invention.

### Description of preferred embodiments

The invention will be further explained by the following examples. The examples demonstrate that the methods of the present invention apply to the legislation for manual techniques of blood sampling. The platform of the invention guarantees a full traceability and security by means of bar code labels applied on the scanned cassettes and/or carrier plates. The software analyses the scanned cassettes and/or carrier plates and brings the scanned image of each colomn and/or each cup to the screen for interpretation. After validation the images and results are archived in the system.

### Example 1: ABO/RH (Grifols) and Groupe Adulte (Opaline)

This is an example of a blood analysis performed using two techniques, i.e. test tube (method Grifols cassette) and carrier plate (in this example of the software screenshots method Opaline is to be interpreted as the carrier plate technique of the invention).

### Step 1: Input test sample, patient and cassette or carrier plate.

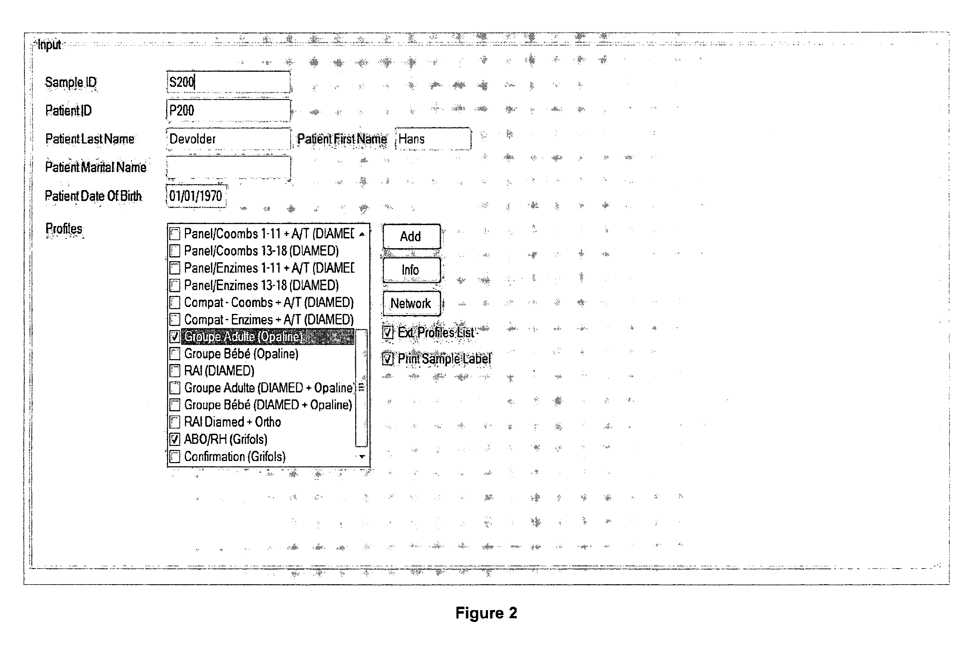

As shown in Figure 2, Sample ID, Patient ID, names and date of birth may be entered by the user or automatically uploaded from a Laboratory Information (Management) System (LI(M)S).
The type of analyses to be performed (see Profiles) is also provided. The profiles list is manifold and corresponds at least to the techniques traditionally used in manual blood typing. Figure 2 indicates that two profiles have been selected: ABO/RH (Grifols) and Groupe Adulte (Opaline). The bracketed items indicate the technique or commercial name of the technique.

One or more profiles can be chosen per test sample.

### Step 2: Distribution (Virtual View)

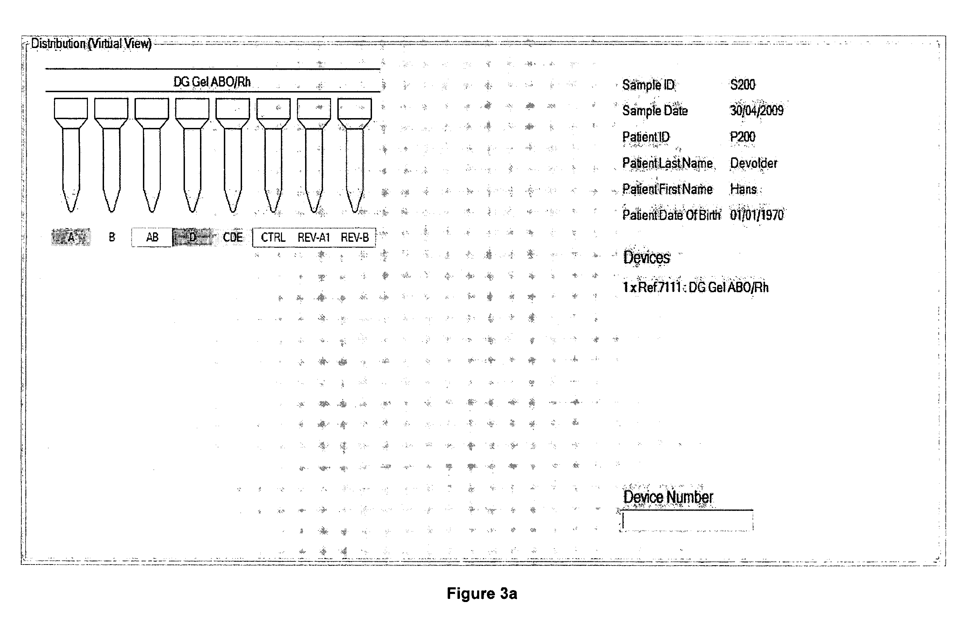

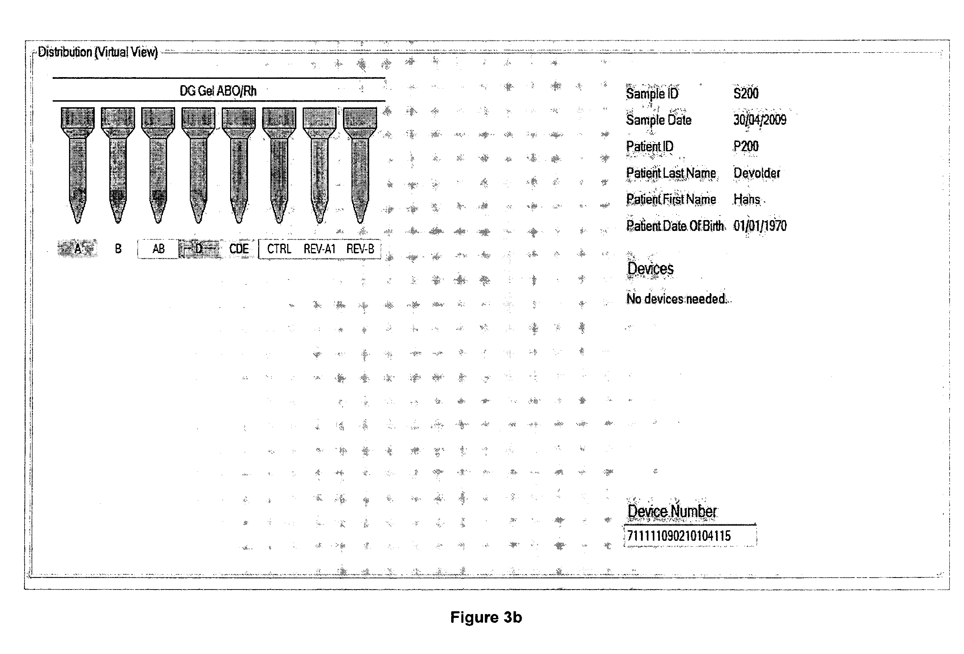

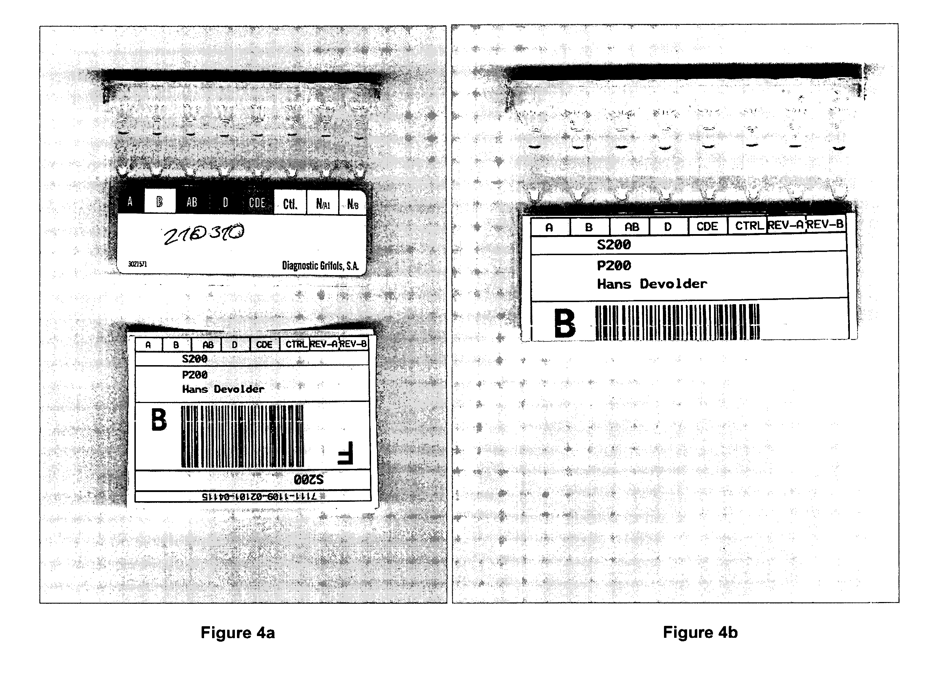

The system will set up the two tests to be performed virtually in an initial phase. Figures 3a and 3b show a virtual view of a first distribution for the ABO/Grifols analyses. It shows an image of the Grifols device to be used. Figure 3b indicates (by coloring) which tubes have to be sampled. In this example all 8 tubes need to be sampled. Other examples could indicate lesser tubes to be sampled. After a device of such type is taken and its Device Number is entered, a label (Figure 4a) is printed and applied to said device (Figure 4b). The label uniquely links the Device ID to the Sample and Patient ID within the system. Additional information may be printed on the label, e.g. Patient Name and identification of test reagents to be added. The label is preferably a barcode label making identification with a handscanner very easy. The label could also be an RFID.
The Device Number may also be scanned in with a handscanner. As such the device type can be double checked, as well as its date of validity.
Note the status bar at the bottom of the screen indiciating the status of the test at each time in the process.

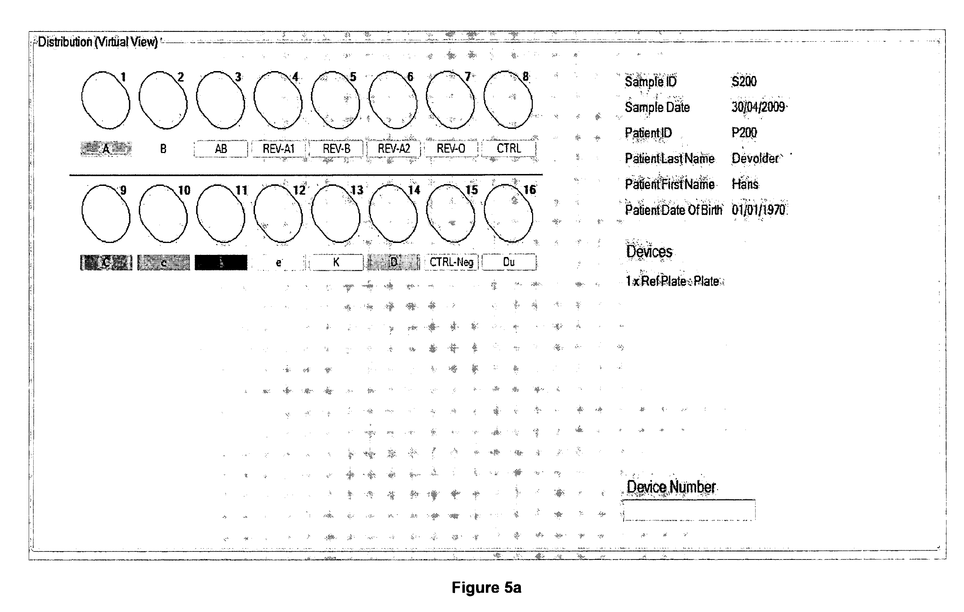

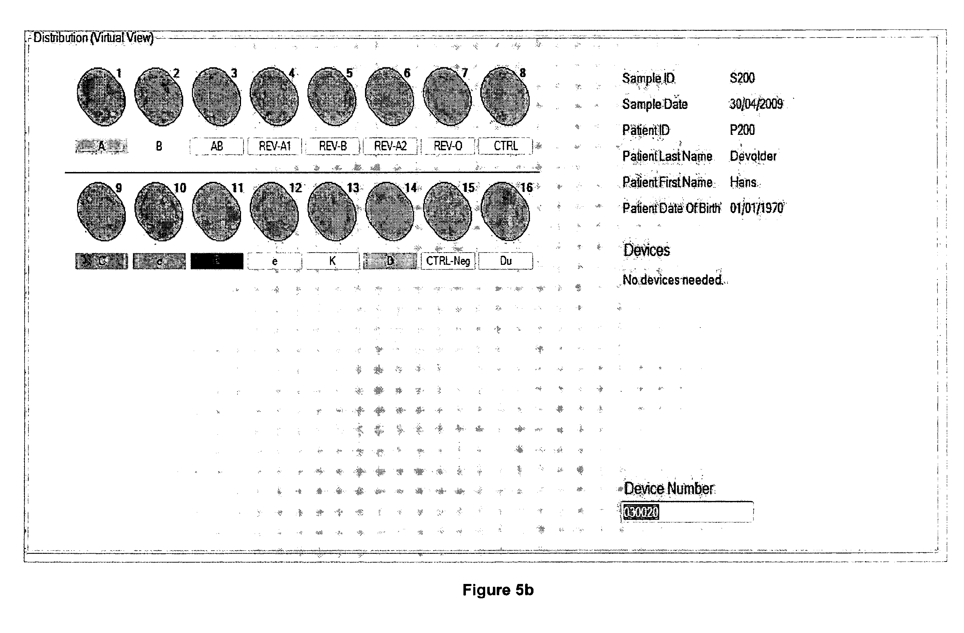

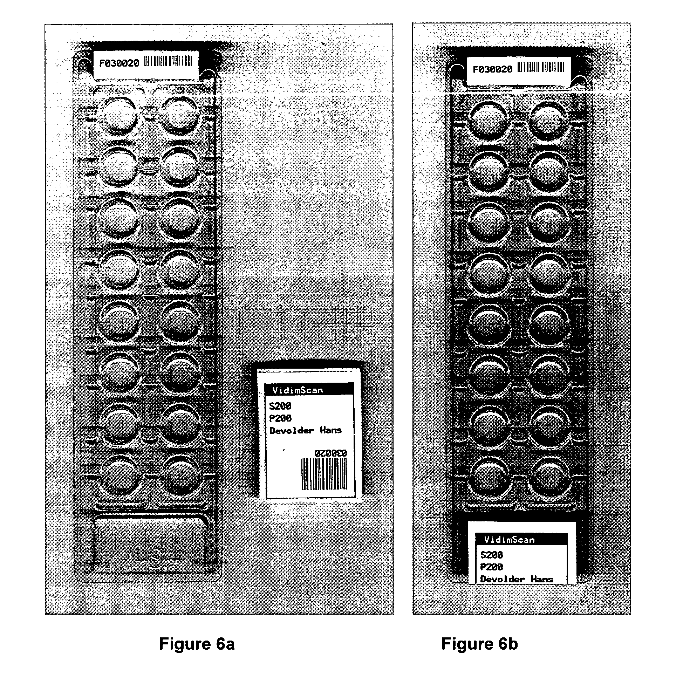

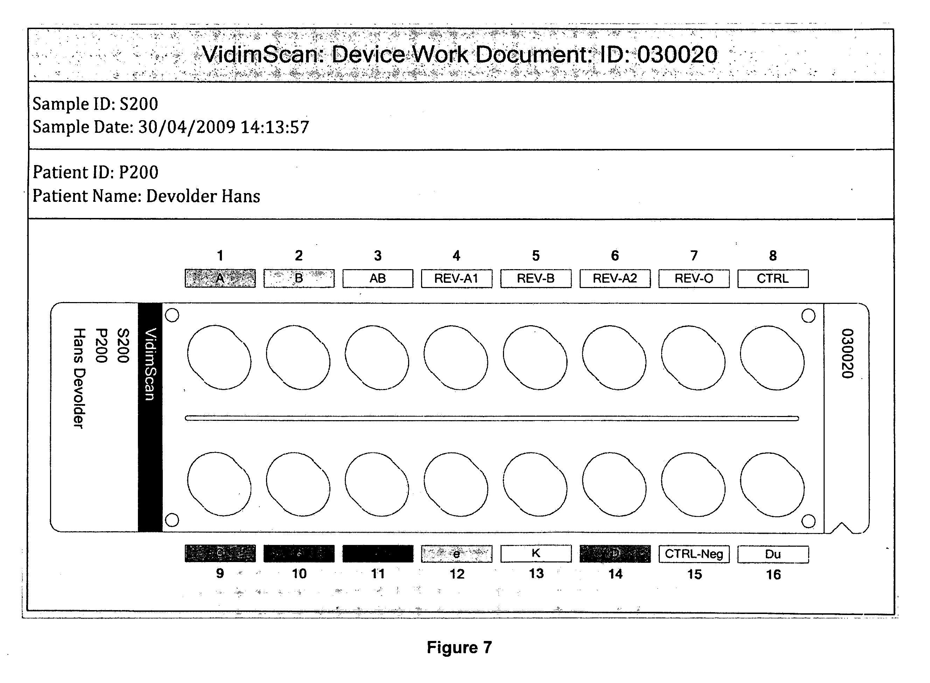

Figures 5a and 5b show the virtual view of a second distribution for the Groupe Adulte (Opaline) analyses. The Opaline analyses is performed in a carrier plate of the invention. Again a Device Number is entered and a label is printed linking the Device, Sample and Patient ID within the system (Figures 6a and 6b).
For carrier plates of the invention, a Device Work Document (Figure 7) is printed indicating the positions of the cups for each reagent. The Device Work Document has a unique ID and may contain additional information, such as e.g. Sample ID, Patient ID and a barcode (not shown on the Figures). The carrier plate can be positioned on the Device Work Document to perform the actual testing. As such, an identification may be applied to the carrier plate identifying the reagents to be added in each of the cups of the carrier plate.

### Step 3: Blood sampling

The actual blood sampling is then performed according to good lab practice within the labeled devices.

### Step 4: Scanning

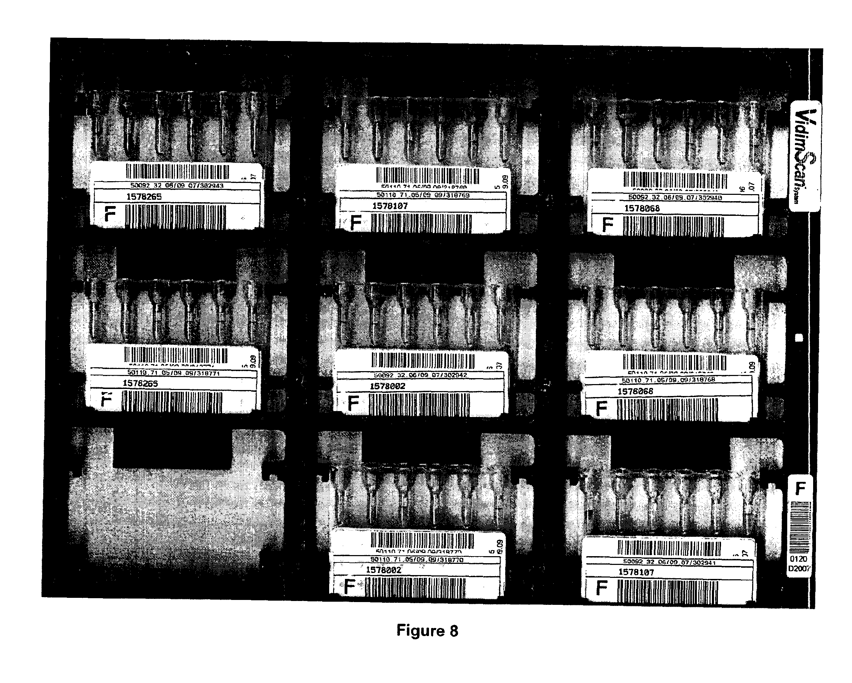

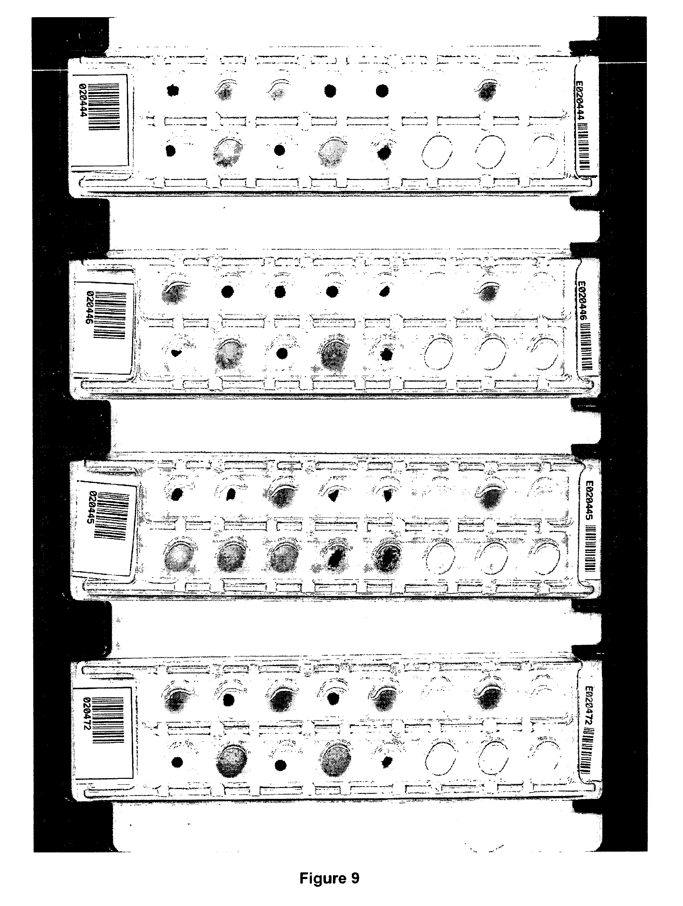

The labeled devices containing the test samples and reactions are then scanned as shown in Figure 8 for a device of type Grifols, and in Figure 9 for a carrier plate device of the invention. Up to nine 6-column cassettes can be scanned in one go, and up to four carrier plates.

For this purpose a dedicated vertical scanner and holding means is provided for the cassette technique, and a dedicated horizontal scanner and holding means for the carrier plate technique. The holding frame of the latter provides unique indents for not misplacing the carrier plates, as can be seen on the top right corner of the four carrier plates of Figure 9. Another embodiment of a holding frame could position or shape the indents differently, as long as the carrier plate is positioned only in one way in the holding frame and the software takes account hereof.

### Step 5: Technical Validation (Physical View)

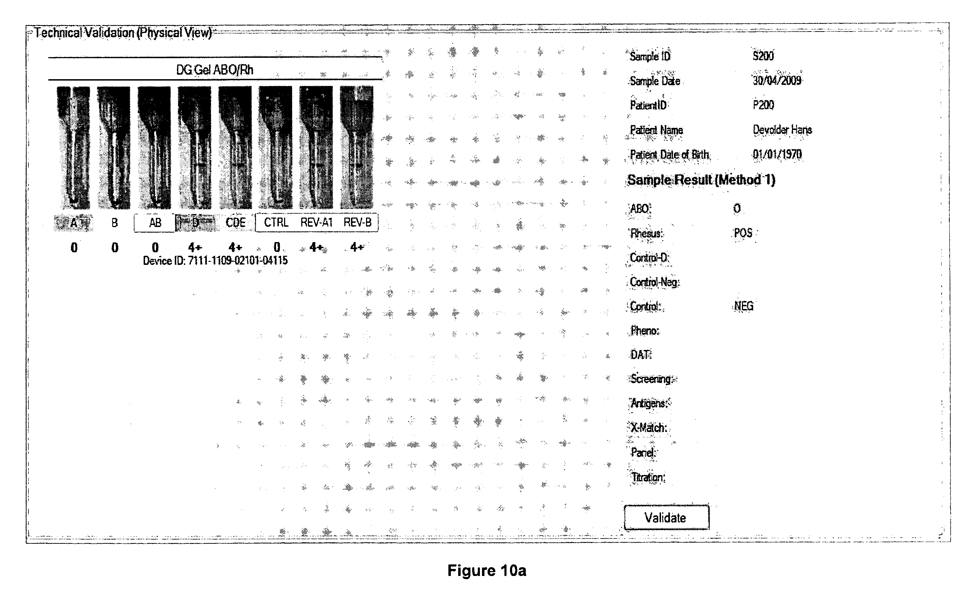

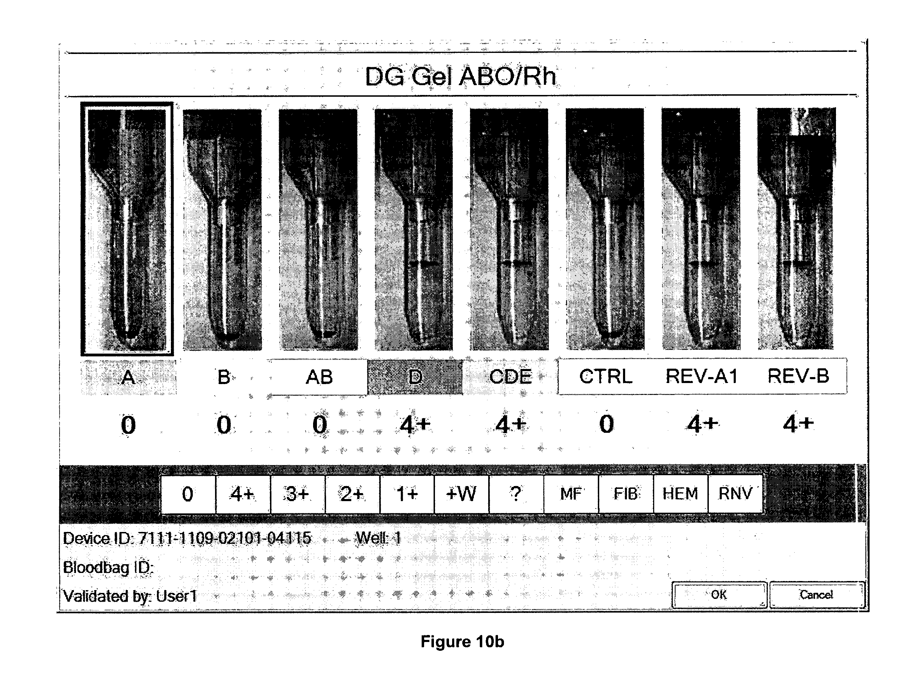

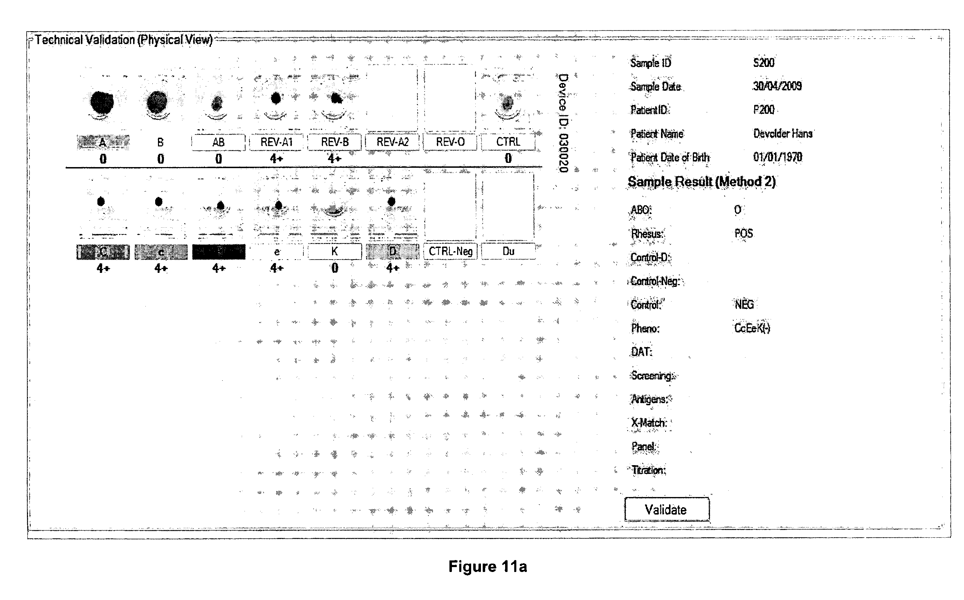

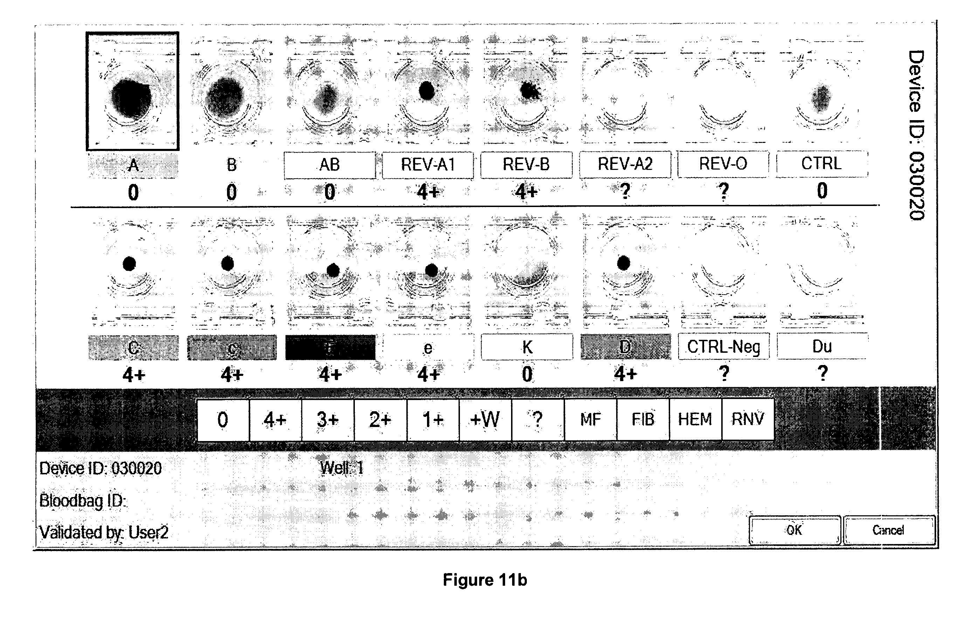

Figure 10a illustrates the scanned image of the Grifols cassette and Figure 11a that of the carrier plate. The lab technician may enter the interpreted results by hand for each tube or cup, as shown in the screenshots of Figures 10b and 11b. The system will check whether the result is not incompatible within this step, i.e. within the Grifols or within the Opaline setup. The system may also automatically detect analyses results by means of a dedicated expert system.

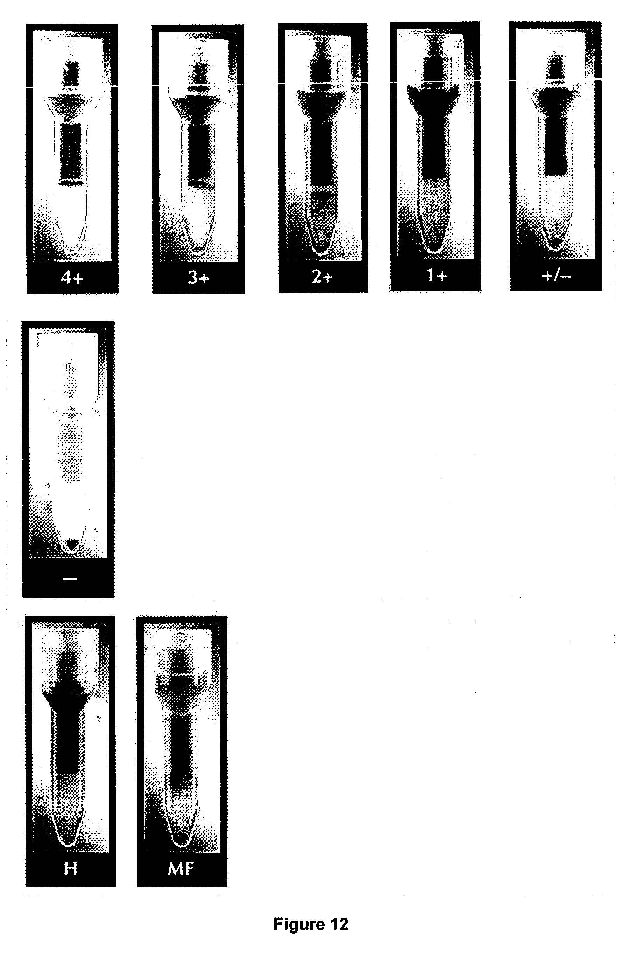

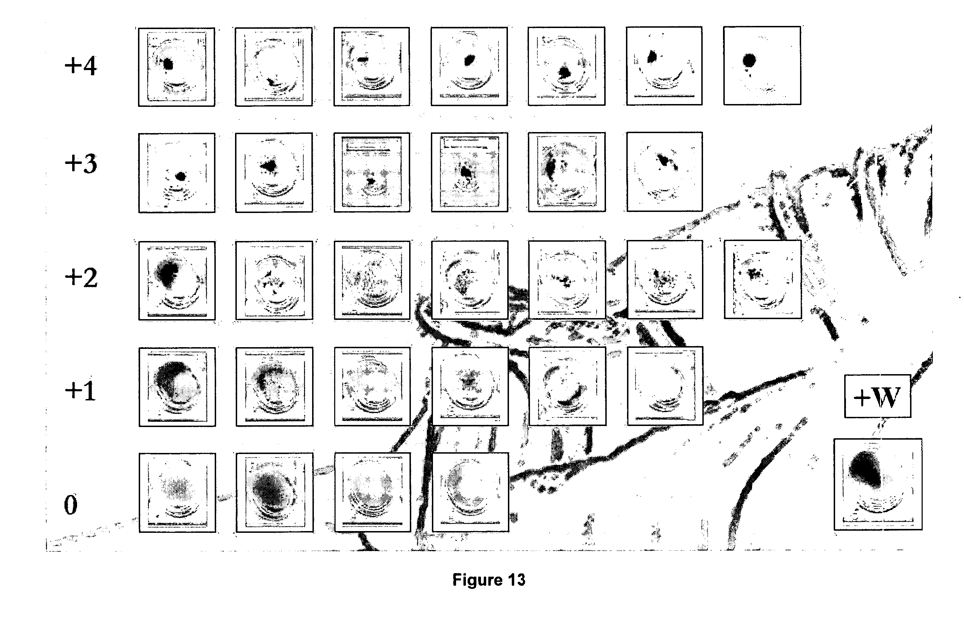

Figures 12 and 13 illustrate the graduation of results for a cassette and a carrier plate technique.

The lab technician finally validates the results using the Validate button on the screen.

### Step 6: Biological Validation (Physical View)

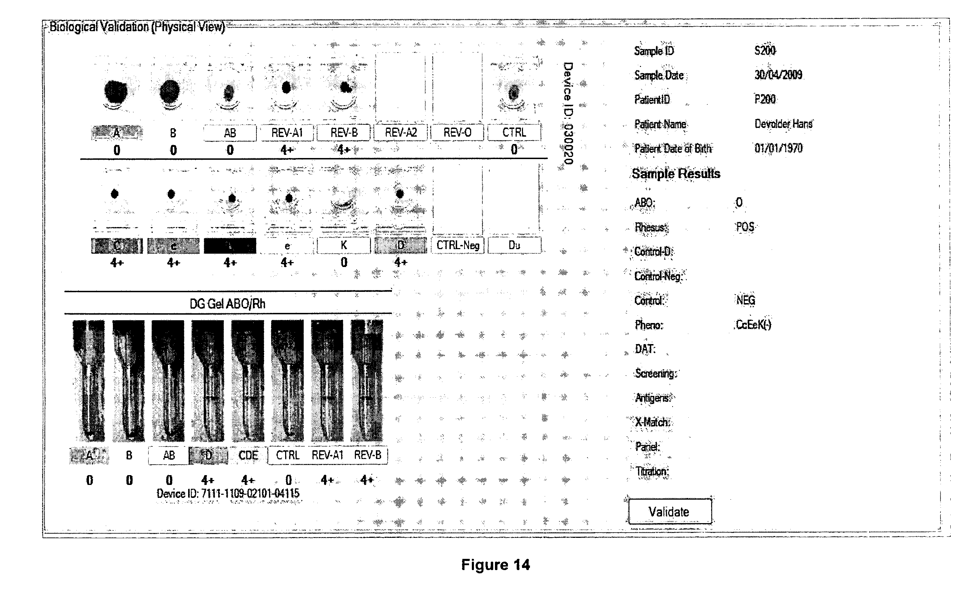

Finally a biological validation step is required, preferably by a lab responsible, to validate and check the obtained sampling results. Figure 14 illustrates a physical view of a full sample test ready for biological validation. The results for each tube or cup can still be changed at the stage before final biological validation. The biological validation step makes a consensus over all sampling results for one patient and one sample ID. Possible discrepancies can be detected and restored accordingly.

Where in step 5 a control check is made for incompatibility within one technical test, step 6 controls incompatibilities over the different sampling results for a patient. The software may also check previous patient results, if any, for possible antecedents or indiscrepancies. If an incompatibility is detected, the result of that cup/tube is invalidated. This may be overruled with the logging of valid comments with a reason why this was done. All these steps are traceable within the system.

### Step 7: Reporting

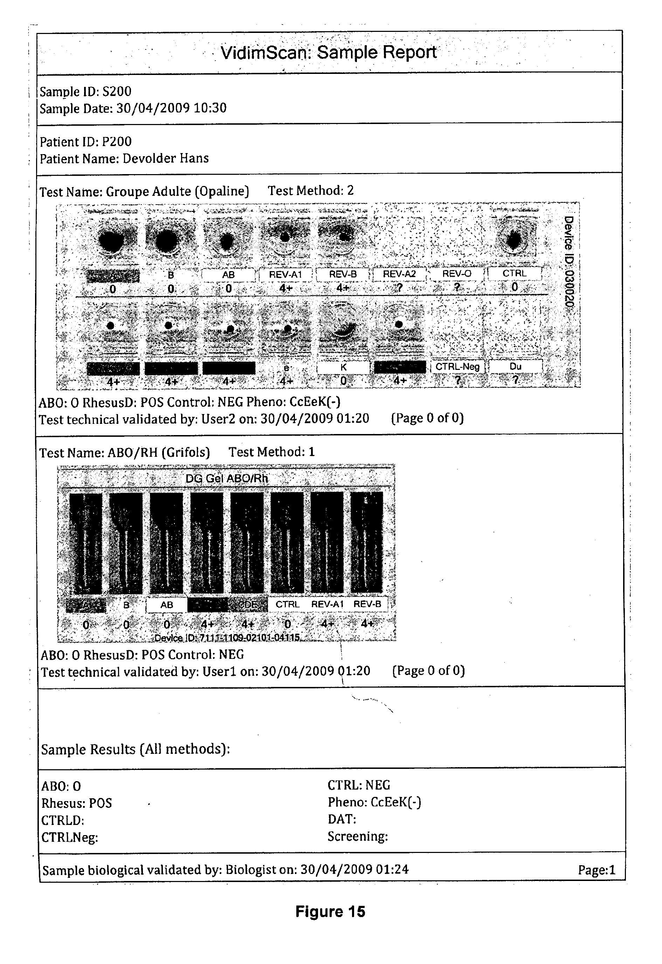

Figure 15 shows a Sample Report or a printout of the results of the sampling and validation.

If secured, the sampling report can be transmitted online to other laboratoria or even to doctors or hospitals. Evenly so, if sufficiently secure, a direct online access to a LIS could be provided.

### Step 8: Archiving and traceability

Finally all of the above information is stored and archived into the system, where all results, linked to samples and patients, can be retrieved and traced at any time and any step of the procedure.

### Example 2: Other combinations of carrier plate and cassette

In the previous example a cassette and a carrier plate technique is combined in one analysis. Other examples could include two or more carrier plates, each for specific tests, or providing double testing, e.g. each with different reagents. As can be made up from the Profiles list, multiple combinations of tests are possible and may be set up by the system allowing a controlled sampling as well as a traceable resulting. New profiles can easily be defined and added to the software. In yet another example one technique (test tube) may be used by means of different methods (e.g. Diamed and Ortho cassettes) for analysing e.g. ABO (Diamed), Pheno (Diamed) and RAI (Ortho). The steps are similar to those described above.

### Example 3: Coagulation device for the carrier plates according to the invention

The present invention provides a coagulation device for the carrier plates of the invention. The coagulation device concentrates the blood sample (and reagent) within the center of the cups to allow coagulation. The device avoids false positives (concentration of non-coagulated blood cells) by keeping said blood cells in suspension. To this purpose, the device generates a trembling movement in a circular translational plane of the carrier plate, with a certain frequence and amplitude. This trembling movement causes a flow of blood cells within the cups of the carrier plate, allowing blood particles with a certain dimension to concentrate in the center of the cup. Non-coagulated blood cells stay in suspension.

Relative large volumes of blood sample are used in the test tube and opaline technique, allowing a manual agitation movement for coagulation purposes. This is not the case for the carrier plate technique of the present invention, whereby much smaller volumes (approx. 50µl blood en 50µl reagent) are used. Agitation by manual movement does not obtain the required result for a good coagulation reaction.

An agglutination reaction within the cups of the carrier plate according to the invention may be obtained as follows. A blood sample and appropriate reagents are pipeted directly into the cups. The carrier plate is then centrifuged using a dedicated coagulation device. In one embodiment the carrier plate is fixed into the centrifuging device by means of spirals bringing over a gentle but firm centrifuging movement to the carrier plate. The movement is such that the mixture is stirred without spilling. After about ten minutes of centrifuging, the stirring movement is changed in opposite direction so as to spread the mixture. Thereafter an agglutination reaction may or may not be observed.

The centrifuging within a carrier plate of the invention combines the advantages of a tube technique (centrifuging a suspension) in an opaline-like plate technique. The added effect is the scanning, storing and traceability of the manual blood typing result.

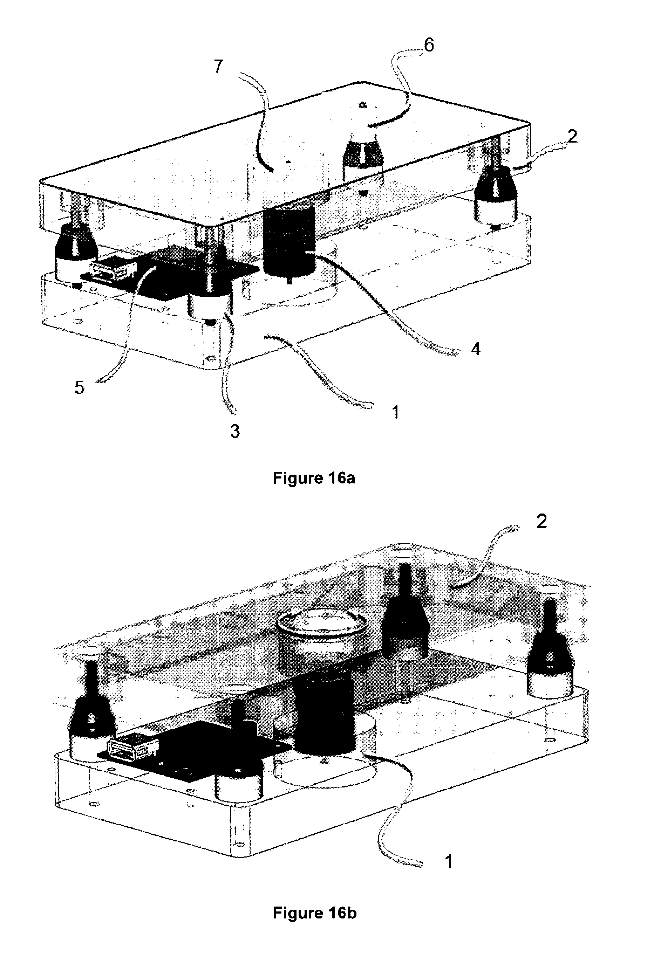

Figure 16a and 16b illustrates a preferred embodiment of a coagulation device according to the invention, comprising two parts: a fixed part (1) for the basis of the device, and a moving part (2) that produces the trembling movement of a carrier plate applied to it (6). The upper moving part is connected to the lower base part by means of four dampers (3) to stabilise the device. The damper is preferably manufactured from an elastomer, such as rubber or silicone. The dampers also absorb the excessive trembling energy and avoid resonance of the coagulation device.

A DC motor (4) controlled by a motor controller (5) provides the trembling movement. The motor, having an excentric mass on the vertically placed drive shaft, is positioned at the center of the device for reasons of generating a pure circular translational movement. For the same reason, the center of mass of the motor is positioned as close as possible to the center of the total trembling mass (7).

Optimal results are obtained by an optimal frequence and amplitude of the trembling movement. The amplitude is dependent on the size of the excentric mass. Replacing the mass by another size mass gives other amplitudes. The frequence is determined by the rotation speed of the motor. Changing the rotation speed can alter the frequence. The rotation speed should remains constant during trembling, as well as during the lifetime of the device. Therefor a brushless DC motor with frequence control is preferably used.

### Example 4: Incubator for the carrier plates according to the invention

In another embodiment the present invention provides an incubator for controlling the temperature of some or all individual cups of the carrier plate.

The coagulation device as described above may further comprise temperature control means for keeping some or all cups of the carrier plate at a certain temperature. In one embodiment the temperature of the four first/last cups (2x2) needs to be fixed at about 4ºC, in another embodiment at about 22ºC, irrespective of roomtemperature. The other cups need to be kept at roomtemperature. The four cups are typically for Rev-A, Rev-B, Rev-O and Rev-AB tests.

In one embodiment the temperature control means are Peltier elements provided for each cup individually (not shown on the Figure). The Peltier element of the four cups has a higher thermic capacity than the Peltier element of the other cups, because a stronger cooling down is required. Electronic temperature control is provided by thermocouples integrated in the Peltier elements.

Other embodiments of a coagulation device are within the scope of the invention.

## Claims

1. A method for automated storing and tracing of a manual blood typing analyses at least partly performed in one or more carrier plates comprising a plurality of cups, said method comprising the steps of:
a. applying a patient ID and a sample ID to said one or more carrier plates;
b. providing a mixture of blood sample and a suitable reagent in one or more cups of said carrier plates;
c. digitizing an image of said carrier plates;
d. interpreting an agglutination reaction within said one or more cups of said carrier plates;
e. storing said patient ID, sample ID, image and interpretation in a automated system.

2. The method of claim 1 wherein step b. further comprises incubating said mixture in said one or more carrier plates.

3. The method of any of claims 1 or 2 wherein the mixture of blood sample and a suitable reagent of step b. is first mixed and optionally incubated in a test tube.

4. The method of any of the previous claims wherein said manual blood typing analyses is partly performed in one or more carrier plates and partly in one or more cassettes, said method further comprising the steps of:
a. applying said patient ID and said sample ID to said cassettes;
b. providing a mixture of blood sample and a suitable reagent in one or more tubes of said cassettes, *and optionally incubating said mixture;*
c. digitizing an image of said cassettes;
d. interpreting an agglutination reaction within said one or more tubes;
e. storing said patient ID, sample ID, image and interpretation in an automated system.

5. The method of any of the previous claims wherein step d. comprises the step of technically validating said interpretation of said blood typing analyses.

6. The method of any of the previous claims further comprising the step of biologically validating all interpretations for said patient ID and said sample ID.

7. The method of any of the previous claims further comprising the step of printing a sample report.

8. The method of any of the previous claims wherein said patient ID and sample ID are linked to said carrier plate or cassette by means of a barcode label.

9. The method of any of the previous claims wherein the step of digitizing is provided by an imaging device.

10. The method of claim 10 wherein said imaging device is a scanner.

11. The method of claim 11 wherein said scanner comprises holding means applied to the scanner surface for holding one or more carrier plates.

12. The method of claim 11 wherein said holding means and said one or more carrier plates further comprise interconnecting means for holding said one or more carrier plates in a unique position within said holding means.

13. The method of any of the previous claims wherein said carrier plate comprises 16 cups.

14. The method of any of the previous claims, further comprising the steps of:
a. selecting a stored blood typing analyses;
b. tracing one or more of the steps performed within said blood typing analyses.
